(19) Europäisches Patentamt
European Patent Office
Office européen des brevets

(11) Veröffentlichungsnummer : **0 023 976**
**B1**

(12) # EUROPÄISCHE PATENTSCHRIFT

(45) Veröffentlichungstag der Patentschrift : **08.02.84**

(51) Int. Cl.³ : **C 07 C 127/22**, A 01 N 47/34, B 27 K 3/38

(21) Anmeldenummer : **80104035.3**

(22) Anmeldetag : **12.07.80**

(54) **N-Aryl-N'-acryloyl-ureide, Verfahren zu ihrer Herstellung sowie ihre Verwendung als Mikrobizide.**

(30) Priorität : **24.07.79 DE 2929863**

(43) Veröffentlichungstag der Anmeldung : **18.02.81 Patentblatt 81/07**

(45) Bekanntmachung des Hinweises auf die Patenterteilung : **08.02.84 Patentblatt 84/06**

(84) Benannte Vertragsstaaten : **AT BE CH DE FR GB IT LI SE**

(56) Entgegenhaltungen :
**DE-A- 1 643 723**
**FR-A- 2 415 962**
**GB-A- 1 221 116**
**US-A- 3 219 644**
**I. Organic Chemistry 23, 644-645(1958)**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Paulus, Wilfried, Dr.**
**Deswatinesstrasse 90**
**D-4150 Krefeld 1 (DE)**
Erfinder : **Genth, Hermann, Dr.**
**Am Heckerhof 60**
**D-4150 Krefeld 1 (DE)**

**EP 0 023 976 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

N-Aryl-N'-acryloyl-ureide, Verfahren zu ihrer Herstellung sowie ihre Verwendung als Mirkobizide

Die Erfindung betrifft ein Verfahren zur Herstellung von neuen N-substituierten Acrylsäureureiden, ein Verfahren zu ihrer Herstellung sowie ihre Verwendung als Mikrobizide.

Es ist aus Chem. Ber. *101,* 2419 bis 2425 (1968) bekannt, daß Harnstoff in heterogener Phase in inerten Lösungsmitteln mit äquimolaren Mengen Acrylsäurechlorid eine Additionsverbindung bildet, die mit Hilfe einer Base zu einem Acrylsäureureid dehydrohalogeniert wird.

Die Herstellung von N-Phenyl-N'-acryloylharnstoff durch Umsetzung von Phenylisocyanat in einem Lösungsmittel, wie o-Dichlorbenzol, mit Acrylsäureamid und in Gegenwart von Hydrochinon als Polymerisationsverzögerer wird in dem DP 888 316 beschrieben.

Durch Umsetzung von Acryl-isocyanaten mit Anilin bzw. mit Toluidin oder Xylidin, erhält man ebenfalls N-Phenyl-N'-acryloyl-ureide bzw. die entsprechenden methylsubstituierten Verbindungen (Ber. (*84,* 4 bis 12 (1951)).

In der DE-AS 16 43 723 wird die biozide Wirkung von Acrylsäureureid gegen Aspergillus niger beschrieben. Gegenüber anderen Pilzen besteht jedoch nur eine unbefriedigende Wirksamkeit.

Aus der GB 12 21 116 sind N-Acyl-N'-(halogenierte-aryl)-ureide bekannt, die jedoch gegenüber Pilzen und Bakterien nur wenig wirksam sind.

In der am Prioritätstag der vorliegenden Anmeldung unveröffentlichten DE-AS 29 04 309 und den äquivalenten Anmeldungen in Großbritannien und Frankreich werden ebenfalls N-Aryl-N'-acryloyl-ureide beansprucht. Zur Abgrenzung von diesen älteren Anmeldungen werden für die Länder Deutschland, Großbritannien und Frankreich eingeschränkte Ansprüche vorgelegt.

Es wurde ein Verfahren zur Herstellung von N-Aryl-N'-acryloyl-ureiden gefunden, nach dem auch die neuen N-Aryl-N'-acryloyl-ureide hergestellt werden können.

Das erfindungsgemäße Verfahren zur Herstellung von N-Aryl-N'-acryloyl-ureiden der Formel

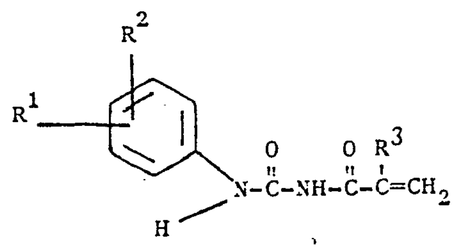

(I)

worin

$R^1$ und $R^2$ gleich oder verschieden sind und Wasserstoff, gegebenenfalls durch Halogen substituiertes Alkyl oder Halogen bedeuten, und

$R^3$ Wasserstoff oder ein geradkettiger oder verzweigter Alkylrest bedeuten, ist dadurch gekennzeichnet, daß man Arylharnstoffe der Formel

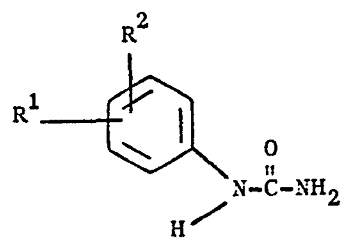

(II)

worin

$R^1$ und $R^2$ die oben genannte Bedeutung haben mit einem β-Chlor-propionylchlorid der Formel

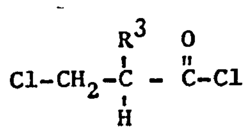

(III)

worin

$R^3$ die obengenannte Bedeutung hat, umsetzt und das entstandene N-Aryl-N'-β-chlorpropionylureid nachfolgend mit einer Base bei erhöhter Temperatur dehydrohalogeniert.

Alkyl kann ein geradkettiger oder verzweigter Kohlenwasserstoffrest mit 1 bis 12 Kohlenstoffatomen sein. Bevorzugt ist ein niederer Alkylrest mit etwa 6 Kohlenstoffatomen. Beispielsweise sei Methyl und Ethyl genannt.

Der Alkylrest kann durch Halogen, Fluor, Chlor, Brom oder Jod, bevorzugt Fluor oder Chlor, substituiert sein.

Halogen kann Fluor, Chlor, Brom oder Jod, bevorzugt Fluor, Chlor oder Brom, bedeuten.

Das erfindungsgemäße Verfahren kann beispielsweise anhand der folgenden Reaktionsgleichungen erläutert werden :

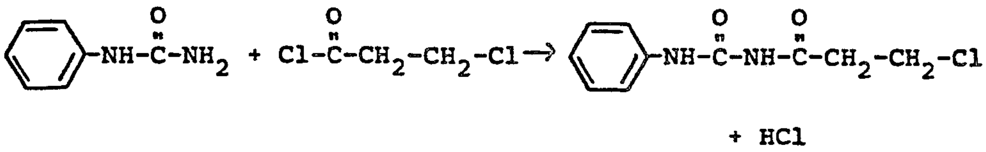

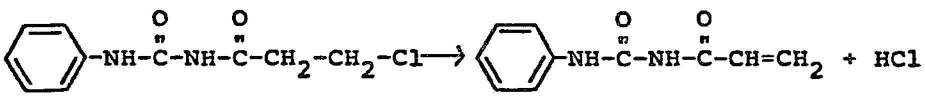

Arylharnstoffe für das erfindungsgemäße Verfahren sind an sich bekannt und können beispielsweise durch Umsetzung von Phenylisocanat und Ammoniak hergestellt werden.

Bevorzugt für das erfindungsgemäße Verfahren sind Arylharnstoffe der Formel

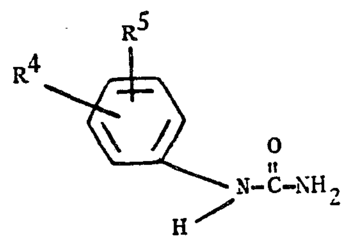

(IV)

worin

$R^4$ und $R^5$ gleich oder verschieden sind und gegebenenfalls durch Halogen substituiertes Alkyl, oder Halogen bedeuten. Sie werden insbesondere mit β-Chlorpropionylchlorid umgesetzt.

Beispielsweise seien die folgenden Arylharnstoffe genannt : Phenylharnstoff, p-Methyl-phenylharnstoff und p-Chlor-phenylharnstoff.

β-Chlorpropionylchloride der Formel (III) sind an sich bekannt und können beispielsweise durch Umsetzung von β-Chlorpropionsäure mit Phosphoroxychlorid hergestellt werden.

Beispielsweise seien die folgenden β-Chlor-propionylchloride der Formel (III) genannt : β-Chlorpropionylchlorid, 2-Methyl-β-chlor-propionylchlorid, 2-Ethyl-β-chlor-propionylchlorid.

Bevorzugtes β-Chlor-propionyl-chlorid der Formel (III) für das erfindungsgemäße Verfahren ist das β-Chlor-propionylchlorid.

Die erfindungsgemäße Umsetzung von Arylharnstoffen mit einem β-Chlor-propionylchlorid wird im allgemeinen bei erhöhter Temperatur durchgeführt. Bevorzugt arbeitet man im Temperaturbereich von 50 bis 150 °C, insbesondere bevorzugt im Temperaturbereich von 75 110 °C.

Die Umsetzung wird im allgemeinen bei Normaldruck durchgeführt ; es ist jedoch auch möglich, die Umsetzung bei einem Unter- oder Überdruck durchzuführen. Die Mengen der Reaktanten sind variabel. Im allgemeinen setzt man 1 bis 2 Mol, bevorzugt 1,1 bis 1,3 Mol, β-Chlorpropionylchlorid, bezogen auf 1 Mol des Arylharnstoffs, ein.

Der erfindungsgemäße Verfahrensschritt wird im allgemeinen in einem Lösungsmittel durchgeführt. Im allgemeinen wählt man aprotische, polare Lösungsmittel wie chlorierte Kohlenwasserstoffe, beispielsweise Ethylenchlorid, Ether, wie Dimethylether, Diethylether, Dimethoxyethan, oder Ketone, wie Aceton.

Man setzt im allgemeinen das Lösungsmittel in einem Überschuß ein.

Bei der erfindungsgemäßen Umsetzung von Arylharnstoffen mit einem β-Chlor-propionylchlorid entstehen im wesentlichen N-Aryl-N'-β-chlorpropionylureide der Formel

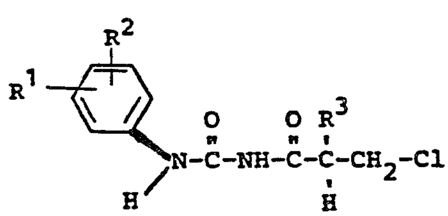

(V)

worin

$R^1$, $R^2$ und $R^3$ die obengenannten Bedeutungen haben.

Für die weitere erfindungsgemäße Umsetzung der N-Aryl-N'-β-chlorpropionylureide ist es nicht erforderlich, diese nach dem ersten Reaktionsschritt zu isolieren.

Selbstverständlich ist es jedoch auch möglich, beim zweiten Reaktionsschritt zuvor isoliertes oder ein auf andere Weise hergestelltes N-Aryl-N'-β-chlorpropionylureid erfindungsgemäß zu dehydrohalogenieren.

Im zweiten Reaktionsschritt des erfindungsgemäßen Verfahrens wird das Reaktionsprodukt des ersten Reaktionsschrittes, also das N-Aryl-N'-β-chlorpropionylureid, mit einer Base bei erhöhter Temperatur dehydrohalogeniert.

Basen für das erfindungsgemäße Verfahren können beispielsweise tertiäre Amine, bevorzugt Triethylamin, sein.

Die Base wird im allgemeinen im Überschuß eingesetzt. Zweckmäßigerweise kann man 1 bis 2 Mol, bevorzugt 1,1 bis 1,5 Mol, der Base bezogen auf 1 Mol des eingesetzten Arylharnstoffes, bzw. des N-Aryl-N'-β-chlorpropionylureids, einsetzen.

Der zweite Schritt des erfindungsgemäßen Verfahrens wird im allgemeinen ebenfalls bei erhöhter Temperatur durchgeführt. Bevorzugt arbeitet man im Temperaturbereich von 35 bis 100 °C, insbesondere bevorzugt im Temperaturbereich von 60 bis 90 °C.

Die Behandlung des Reaktionsproduktes des ersten Verfahrensschrittes mit einer Base wird im allgemeinen ebenfalls bei Normaldruck durchgeführt ; es ist jedoch auch möglich, die Behandlung bei Unter- oder Überdruck auszuführen.

Im allgemeinen wird der zweite Schritt des erfindungsgemäßen Verfahrens im gleichen Lösungsmittel wie in dem ersten Schritt durchgeführt. Es ist selbstverständlich auch möglich, das Lösungsmittel zu ändern.

Falls das erfindungsgemäße Verfahren in zwei getrennten Reaktionsschritten durchgeführt wird, wählt man zweckmäßigerweise für den zweiten Reaktionsschritt ein mit Wasser mischbares Lösungsmittel, wie beispielsweise Aceton oder Dioxan, da sich dann die Aufarbeitung leicht durch Fällen mit Wasser durchführen läßt.

Auch im zweiten Reaktionsschritt wird das Lösungsmittel im allgemeinen im Überschuß eingesetzt.

Das erfindungsgemäße Verfahren zur Herstellung von N-Aryl-N'-acryloyl-ureiden kann beispielsweise wie folgt durchgeführt werden :

Ein Arylharnstoff und ein β-Chlor-propionylchlorid werden in einem geeigneten Lösungsmittel und bei erhöhter Temperatur umgesetzt. Bei der Durchführung des erfindungsgemäßen Verfahrens in einem sogenannten Eintopfverfahren, tropft man zu dem erhaltenen Reaktionsgemisch der ersten Reaktionsstufe die Base zu, und isoliert das Reaktionsprodukt in an sich bekannter Weise, beispielsweise durch Abdestillieren des Lösungsmittels, Waschen mit Wasser und Umkristallisation.

Falls man das Reaktionsprodukt des ersten Reaktionsschrittes isoliert, nimmt man dieses in einem Lösungsmittel auf, gibt die Base hinzu und isoliert nach der Umsetzung das erhaltene N-Aryl-N'-acryloyl-ureid beispielsweise wie bei dem Eintopfverfahren.

Nach dem erfindungsgemäßen Verfahren können neue N-Aryl-N'-acryloyl-ureide der Formel

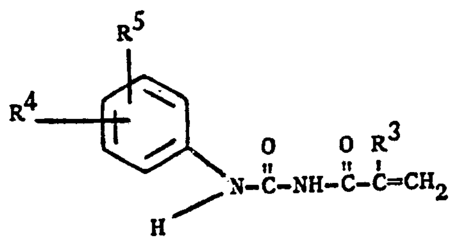

(VI) .

worin

$R^4$ und $R^5$ gleich oder verschieden sind und gegebenenfalls durch Halogen substituiertes Alkyl oder Halogen bedeuten, wobei für den Fall, daß $R^4$ und $R^5$ Methyl bedeutet, das andere von $R^4$ und $R^5$ von Methyl verschieden ist,

$R^3$ Wasserstoff oder ein geradkettiger oder verzweigter Alkylrest bedeutet, hergestellt werden.

Die nach dem erfindungsgemäßen Verfahren hergestellten N-Aryl-N'-acryloyl-ureide der Formel

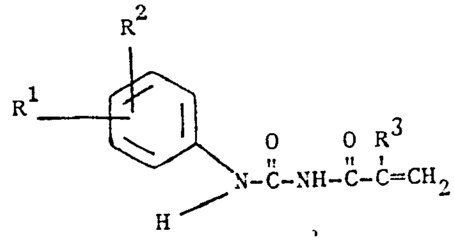

(VII)

worin

$R^1$ und $R^2$ gleich oder verschieden sind und Wasserstoff, gegebenenfalls durch Halogen substituiertes Alkyl oder Halogen bedeuten, wobei für den Fall, daß eines von $R^1$ und $R^2$ Wasserstoff, Methyl oder in 4-Stellung stehendes Chlor bedeutet, das andere von $R^1$ und $R^2$ immer von Wasserstoff verschieden ist, und wobei für den Fall, daß eines von $R^1$ und $R^2$ Methyl bedeutet, das andere von $R^1$ und $R^2$ auch von Methyl verschieden ist, und

$R^3$ Wasserstoff oder ein geradkettiger oder verzweigter Alkylrest bedeutet, sind Verbindungen mit besonders hoher mikrobizider Wirkung. Sie können beispielsweise zum Schutz technischer Materialien gegen einen mikrobiellen Abbau oder eine durch Mikroorganismen bedingte Veränderung der technischen Materialien und zur Bekämpfung von Pflanzenkrankheiten im Pflanzenschutz verwendet werden.

Bevorzugt werden mikrobizide Mittel der Formel

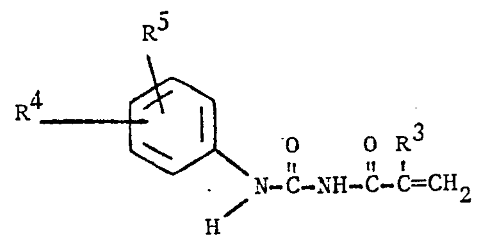

(VIII)

worin

$R^3$ Wasserstoff oder ein geradkettiger oder verzweigter Alkylrest bedeutet, und

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist.

Technische Materialien sind z. B. Klebstoffe, Leime, Papiere und Kartone, Textilien, Leder, Holz, Anstrichmittel, Putze und Gebindeinhalte, die durch mikrobielle Einwirkung geschädigt oder zerstört werden können. Die erfindungsgemäßen Wirkstoffe sind besonders geeignet, Holz gegen mikrobiellen Abbau zu schützen.

Mikroorganismen, die einen Abbau oder eine Veränderung der technischen Materialien bewirken können, sind beispielsweise Bakterien, Pilze, Schleime, Algen und Viren.

Als Bakterien und Pilze seien beispielsweise genannt : Alternaria tenuis, Aspergillus niger, Botrytis cinera, Chaetomium globosum, Cercospora musae, Colletotrichum coffeanum, Coniophora cerebella, Cladosporium herbarum, Penicillium glaucum, Penicillium sasakii, Puccinia recondita, Pullularia pullulans, Phytophora cactorum, Pyricularia oryzae, Pythium ultimum, Rhizopus nigricans, Tilletia caries, Trichoderma viride, Staphylococcus aureus.

Die erfindungsgemäßen Wirkstoffe wirken auch algizid, beispielsweise auf Stichococcus bacillaris, Phormidium foveolarum, Oscillatoria geminata, Euglena gracilis, Phaeodactylum tricornutum, Chlorella pyrenoidosa.

Die erfindungsgemäßen Wirkstoffe werden bevorzugt als Algen- und Schleimbekämpfungsmittel verwendet.

Je nach ihrem Anwendungsgebiet können die erfindungsgemäßen N-Aryl-N'-aryloyl-ureide in die üblichen Formulierungen überführt werden, wie Lösungen, Emulsionen, Suspensionen, Pulver, Pasten und Granulate. Diese können in an sich bekannter Weise hergestellt werden, z. B. durch Vermischen der Wirkstoffe mit einem Streckmittel, d. h. flüssigen Lösungsmitteln und/oder festen Trägerstoffen, gegebenenfalls unter Verwendung von oberflächenaktiven Mitteln, wie Emulgatoren und/oder Dispergiermitteln, wobei beispielsweise im Falle der Benutzung von Wasserstreckmittel, gegebenenfalls organische Lösungsmittel, als Hilfslösungsmittel verwendet werden können.

Die erfindungsgemäßen Wirkstoffe können in den Formulierungen in Mischungen mit anderen bekannten Wirkstoffen vorliegen. Beispielsweise seien die folgenden Wirkstoffe genannt : Benzimidazolyl-alkylcarbamat, Tetramethyl-thiuram-disulfid, N-Fluordichlormethylthiophthalimid und N,N-Dimethyl-N'-phenyl-(N'-fluordichlormethylthio)-sulfamid.

Die Formulierungen enthalten im allgemeinen von 0,1 bis 95 Gew.-% an N-Aryl-N'-acryloyl-ureid als Wirkstoff.

Bevorzugt sind Formulierungen, die von 0,5 bis 90 Gew.-% an Wirkstoff enthalten.

Die Wirkstoffe können als solche, in Form ihrer Formulierungen oder in den daraus bereiteten Anwendungsformen, wie gebrauchsfertige Lösungen, emulgierbare Konzentrate, Emulsionen, Suspensionen, Spritzpulver, Pasten, Löslichpulver, Stäubemittel und Granulate angewendet werden.

Die Anwendungskonzentrationen (Wirkstoffkonzentrationen in anwendungsfertigen Zubereitungen) können in größeren Bereichen variiert werden. Im allgemeinen liegen sie im Bereich von 0,001 und 10 Gew.-%, vorzugsweise im Bereich von 0,01 und 5 Gew.-%.

Da die erfindungsgemäßen N-Aryl-N'-acryloyl-ureide eine starke fungitoxische und bakteriotoxische Wirkung aufweisen und sie Kulturpflanzen in den zur Bekämpfung von Pilzen und Bakterien notwendigen Konzentrationen nicht schädigen, sind sie ebenfalls für den Gebrauch als Pflanzenschutzmittel zur Bekämpfung von Pilz- und Bakterienkrankheiten geeignet.

Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Verbindungen mit bakteriotoxischer Wirkung finden Verwendung gegen phytopathogene Bakterien, z. B. der Gattungen Pseudomonas, Xanthomonas, Erwinia und Corynebacteriaceae.

Die erfindungsgemäßen Wirkstoffe haben ein breites Wirkungsspektrum und können angewandt werden gegen parasitäre Pilze und Bakterien, die oberirdische Pflanzenteile befallen oder die Pflanzen vom Boden her angreifen, sowie gegen samenübertragbare Krankheitserreger.

Eine besonders gute Wirksamkeit entfalten die erfindungsgemäßen Wirkstoffe als Saatbeizmittel gegen den Erreger des Weizensteinbrands (Tilletia caries) und als Blattfungizid gegen den Erreger des Getreiderostes (Puccinia recondita) und gegen Pyricularia oryzae, der eine Blattkrankheit an Reis verursacht.

Bei der Verwendung als Blattfungizide können die Wirkstoffkonzentrationen in den Anwendungsformen in einem größeren Bereich variiert werden. Sie liegen im allgemeinen im Bereich von 0,1 und 0,000 01 Gew.-%, vorzugsweise im Bereich von 0,05 und 0,000 1 Gew.-%.

Bei der Saatgutbehandlung werden im allgemeinen Wirkstoffmengen von 0,001 bis 50 g je kg Saatgut, vorzugsweise 0,01 bis 10 g, je kg Saatgut benötigt.

Die Formulierungen für den Pflanzenschutz enthalten im allgemeinen von 0,1 bis 95 Gew.-% Wirkstoff, vorzugsweise von 0,5 bis 90 Gew.-% Wirkstoff.

Die erfindungsgemäßen N-Aryl-N'-acryloyl-ureide haben vorteilhafterweise eine geringe Phytotoxizität und eine hohe Stabilität gegen Umwelteinflüsse. Sie verändern beispielsweise Farben in technischen Materialien nicht, sind nicht flüchtig und praktisch unlöslich in Wasser.

## Beispiele

### A) Herstellung der N-Aryl-N'-aryloyl-ureide

#### Beispiel 1

Herstellung von N-Phenyl-N'-acryloyl-harnstoff

Zu 2 Mol N-Phenyl-harnstoff in 1,5 l Ethylenchlorid werden bei 60 °C unter Rühren 2,1 Mol β-Chlorpropionylchlorid getropft : anschließend hält man die Reaktionsmischung für 4 Stunden bei Rückflußtemperatur. Beim Abkühlen kristallisiert N-Phenyl-N'-(β-chlorpropionyl)-harnstoff (A) aus. Fp 168 °C ; Ausbeute 422 g = 93 % der Theorie. 0,5 Mol A werden in 750 ml Dioxan unter Erwärmen gelöst und mit 0,55 Mol Triethylamin versetzt ; nach 0,5 Stunden bei 90 °C wird N-Phenyl-N'-acryloylharnstoff mit Wasser ausgefällt. Fp 147 °C Ausbeute 92,4 g = 97 % der Theorie. Für $C_{10}H_{10}N_2O_2$ berechnet : C = 63,1 % H = 5,3 % N = 14,7 % O = 16,9 % ; gefunden : C = 63,15 % H = 5,57 % N = 14,7 % O = 17,1 %.

#### Beispiel 2

1,1 Mol β-Chlorpropionsäurechlorid werden in 750 ml Ethylenchlorid gelöst und mit 1 Mol Phenylharnstoff versetzt. Das Reaktionsgemisch wird auf Rückflußtemperatur (etwa 83 °C) erhitzt und 4, 5 Stunden gerührt. Hierbei fällt β-Chlorpropionsäureureid kristallin aus.

Man kühlt das so erhaltene Reaktionsgemisch auf 70 °C ab und tropft 1,2 Mol Triethylamin zu. Die Temperatur steigt zunächst auf 80 °C an und es bildet sich eine klare Lösung, aus der Kristalle ausfallen. Nach einer Reaktionszeit von 0,5 Stunden bei 80 °C kühlt man ab. Es fällt kristallines N-Aryl-N'-acryloylureid aus, das mit Wasser gewaschen und anschließend getrocknet wird.

Man erhält 135,4 g N-Phenyl-N'-acryloyl-harnstoff (71,2 % d. Th.).

Schmelzpunkt : 151 bis 152 °C.

### B) Anwendungsbeispiele

#### Beispiel 3

N-(p-Chlorphenyl)-N'-acryloyl-harnstoff

Fp 204 bis 208 °C. Für $C_{10}H_9ClN_2O_2$ N ber. : 12,5 % ; N gef. : 12,7 %.

#### Beispiel 4

N-(p-Methylphenyl)-N'-acryloyl-harnstoff

Fp 169 bis 170 °C. Für $C_{11}H_{12}N_2O_2$ N ber. : 13,7 % ; N gef. 13,75 %.

#### Beispiel 5

N-(o-Methylphenyl)-N'-acryloyl-harnstoff

Fp 154 bis 155 °C. Für $C_{11}H_{12}N_2O_2$ N ber. : 13,7 % ; N gef. : 13,75 %.

Beispiel 6

N-(m-Trifluormethylphenyl)-N'-acryloyl-harnstoff
Fp 169 bis 170 °C. Für $C_{11}H_9F_3N_2O_2$ N ber. : 10,9 % ; N gef. : 11,0 %.

Beispiel 7

N-(3,4-Dichlorphenyl)-N'-acryloyl-harnstoff
Fp 188 bis 189 °C. Für $C_{10}H_8Cl_2N_2N_2$ N ber. : 10,8 % ; N gef. : 10,9 %.

Beispiel 8

N-(3-Chlor-4-methylphenyl)-N'-acryloylharnstoff
Fp 177 bis 178 °C. Für $C_{11}H_{11}Cln_2O_2$ N ber. : 11,7 % ; N gef. : 11,7 %.

Beispiel 9

N-(2,6-Disopropylphenyl)-N'-acryloylharnstoff
Fp 215 bis 220 °C. Für $C_{16}H_{22}N_2O_2$ N ber. : 10,2 % N gef. : 10,4 %.

Beispiel 10

N-Phenyl-N'-methacryloylharnstoff
Fp 129 bis 130 °C. Für $C_{11}H_{12}N_2O_2$ N ber. : 13,7 % ; N gef. : 13,9 %.

Beispiel 11

In einen Agar, der aus Bierwürze und Pepton hergestellt wurde, wurde N-(2-methyl)-phenyl-N'-acryloyl-harnstoff jeweils in abgestuften Konzentrationen zwischen 0,000 5 und 0,5 Gew.-% je Versuchsprobe eingearbeitet. Nach dem Erstarren des Agars erfolgte die Kontamination der so hergestellten Agarproben mit Reinkulturen verschiedener Testpilze (s. Tabelle 1).

Nach zweiwöchiger Lagerung bei 28 °C und 60 bis 70 % relativer Luftfeuchtigkeit wurde ausgewertet. In Tabelle 1 ist als minimale Hemmkonzentration (MHK) die geringste in einer Agarprobe enthaltene Konzentration der o. g. Substanz angegeben, bei der keinerlei Bewuchs durch die verwendete Art erfolgte.

Tabelle 1

| Testorganismen | MHK-Werte von N-(2-methyl)-phenyl-N'-acryloyl-harnstoff in % |
|---|---|
| Alternaria tenuis | 0,01 |
| Chaetomium globosum | 0,004 |
| Coniophora cerebella | 0,000 2 |
| Lentinus tigrinus | 0,002 |
| Penicillium glaucum | 0,015 |
| Polyporus versicolor | 0,01 |
| Pullularia pullulans | 0,004 |
| Sclerophoma pityophila | 0,001 |
| Trichoderma viride | 0,02 |

Beispiel 12

Zur Ermittlung der MHK einer weiteren erfindungsgemäßen Substanz wurde wie in Beispiel A beschrieben verfahren ; als Testorganismen dienten holzzerstörende Pilze (Basidiomyceten). Die Ergebnisse sind in Tabelle II zusammengefaßt.

(Siehe Tabelle 2 Seite 8 f.)

Tabelle 2

| Verbindung | MHK in Gew.-% Coniophora cerebella | Lentinus tigrinus | Polyporus versicolor | Sclerophoma pityophila |
|---|---|---|---|---|
| N-(4-Methyl)-phenyl-N'-acrylyl-harnstoff | 0,000 1 | 0,001 5 | 0,005 | 0,000 15 |

**Ansprüche** (für die Vertragsstaaten : DE, FR, GB)

1. Verfahren zur Herstellung von N-Aryl-N'-acryloylureiden der Formel

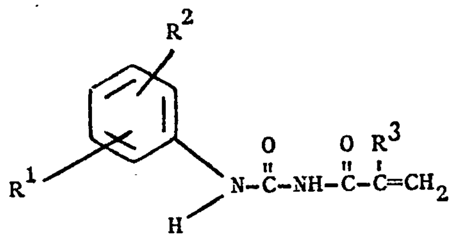

worin

$R^1$ und $R^2$ gleich oder verschieden sind und Wasserstoff, gegebenenfalls durch Halogen substituiertes Alkyl oder Halogen bedeuten, und

$R^3$ Wasserstoff oder einen geradkettigen oder verzweigten Alkylrest bedeutet, dadurch gekennzeichnet, daß man Arylharnstoffe der Formel

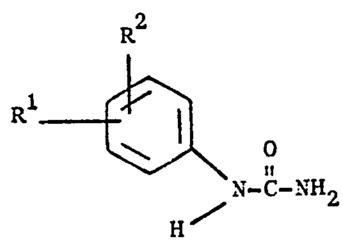

worin

$R^1$ und $R^2$ die obengenannte Bedeutung haben, mit einem β-Chlor-propionylchlorid der Formel

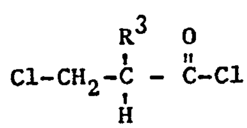

worin

$R^3$ die obengenannte Bedeutung hat, umsetzt und das entstandene N-Aryl-N'-β-chlorpropionylureid nachfolgend mit einer Base bei erhöhter Temperatur dehydrohalogeniert.

2. Verfahren nach Anspruch 1, dadurch gekennzeichnet, daß man als Ausgangsstoffe einen Arylharnstoff der Formel

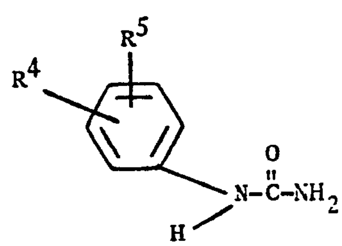

worin

$R^4$ und $R^5$ gleich oder verschieden sind und gegebenenfalls durch Halogen substituiertes Alkyl oder Halogen bedeuten, und β-Chlor-propionylchlorid verwendet.

3. Verfahren nach den Ansprüchen 1 und 2, dadurch gekennzeichnet, daß man als Base ein tertiäres Amin verwendet.

4. Mikrobizides Mittel enthaltend Verbindungen der Formel

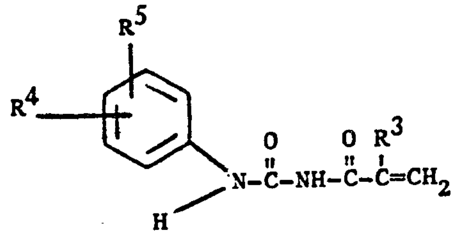

worin

$R^3$ Wasserstoff oder ein geradkettiger oder verzweigter Alkylrest bedeutet, und

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist.

5. Verwendung der mikrobiziden Mittel der Formel

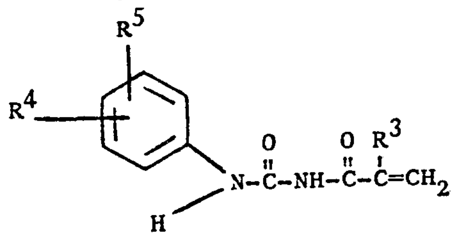

worin

$R^3$ Wasserstoff oder ein geradkettiger oder verzweigter Alkylrest bedeutet, und

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist, zum Schutz von Klebstoffen, Leimen, Papieren und Kartonen, Textilien, Leder, Holz, Anstrichmitteln, Putzen und Gebindeinhalten, die durch mikrobielle Einwirkung geschädigt oder zerstört werden können.

6. Verwendung der mikrobiziden Mittel nach Anspruch 5 zum Schutz von Holz gegen mikrobiellen Abbau.

7. Verwendung der mikrobiziden Mittel nach Anspruch 5 als Algen- und Schleimbekämpfungsmittel.

8. Verwendung der mikrobiziden Mittel der Formel

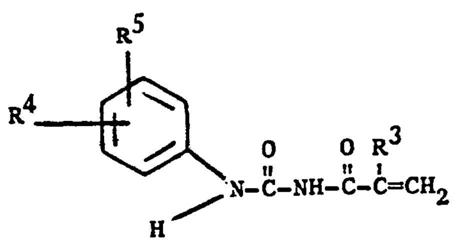

worin

$R^3$ Wasserstoff oder ein geradkettiger oder verzweigter Alkylrest bedeutet, und

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist, im Pflanzenschutz.

9. N-Aryl-N'-acryloyl-ureide der Formel

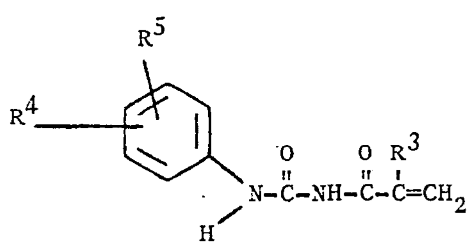

worin

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist, und

$R^3$ Wasserstoff oder ein geradkettiger oder verzweigter Alkylrest bedeutet.

**Ansprüche** (für die Vertragsstaaten : AT, BE, CH, IT, LI, SE)

1. Verfahren zur Herstellung von N-Aryl-N'-acryloylureiden der Formel

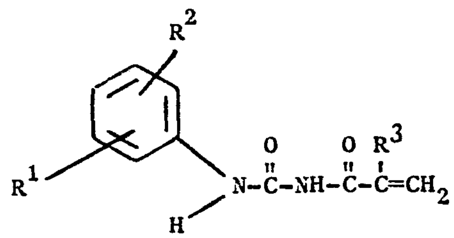

worin

$R^1$ und $R^2$ gleich oder verschieden sind und Wasserstoff, gegebenenfalls durch Halogen substituiertes Alkyl oder Halogen bedeuten, und

$R^3$ Wasserstoff oder einen geradkettigen oder verzweigten Alkylrest bedeutet, dadurch gekennzeichnet, daß man Arylharnstoffe der Formel

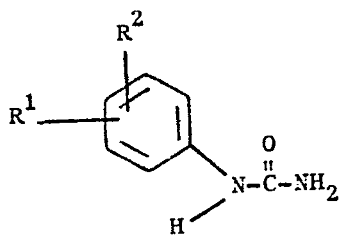

worin

$R^1$ und $R^2$ die obengenannte Bedeutung haben, mit einem β-Chlor-propionylchlorid der Formel

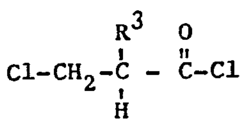

worin

$R^3$ die obengennante Bedeutung hat, umsetzt und das entstandene N-Aryl-N'-β-chlorpropionylureid nachfolgend mit einer Base bei erhöhter Temperatur dehydrohalogeniert.

2. Verfahren nach Anspruch 1, dadurch gekennzeichnet, daß man als Ausgangsstoffe einen Arylharnstoff der Formel

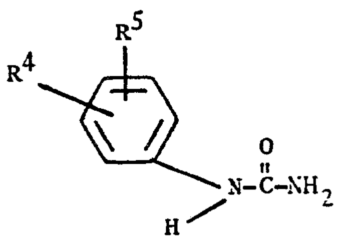

worin

$R^4$ und $R^5$ gleich oder verschieden sind und gegebenenfalls durch Halogen substituiertes Alkyl oder Halogen bedeuten, und β-Chlor-propionylchlorid verwendet.

3. Verfahren nach den Ansprüchen 1 und 2, dadurch gekennzeichnet, daß man als Base ein tertiäres Amin verwendet.

4. Mikrobizides Mittel, enthaltend Verbindungen der Formel

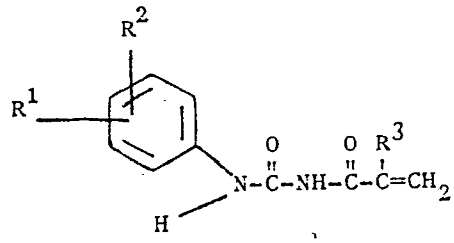

worin

$R^1$ und $R^2$ gleich oder verschieden sind und Wasserstoff, gegebenenfalls durch Halogen substituiertes Alkyl oder Halogen bedeuten, wobei für den Fall, daß eines von $R^1$ und $R^2$ Wasserstoff, Methyl oder in 4-Stellung stehendes Chlor bedeutet, das andere von $R^1$ und $R^2$ immer von Wasserstoff verschieden ist, und wobei für den Fall, daß eines von $R^1$ und $R^2$ Methyl bedeutet, das andere von $R^1$ und $R^2$ auch von Methyl verschieden ist, und

$R^3$ Wasserstoff oder ein geradkettiger oder verzweigter Alkylrest bedeutet.

5. Mikrobizides Mittel nach Anspruch 4, enthaltend Verbindungen der Formel

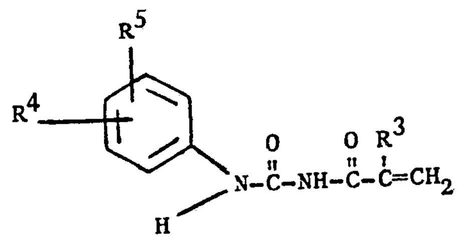

worin

$R^3$ Wasserstoff oder ein geradkettiger oder verzweigter Alkylrest bedeutet, und

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist.

6. Verwendung der mikrobiziden Mittel der Formel

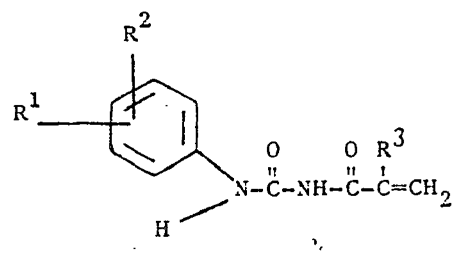

worin

$R^1$ und $R^2$ gleich oder verschieden sind und Wasserstoff, gegebenenfalls durch Halogen substituiertes Alkyl oder Halogen bedeuten, und

$R^3$ Wasserstoff oder ein geradkettiger oder verzweigter Alkylrest bedeutet, zum Schutz von Klebstoffen, Leimen, Papieren und Kartone, Textilien, Leder, Holz, Anstrichmittel, Putze und Gebindeinhalte, die durch mikrobielle Einwirkung geschädigt oder zerstört werden können.

7. Verwendung der mikrobiziden Mittel nach Anspruch 6 zum Schutz von Holz gegen mikrobiellen Abbau.

8. Verwendung der mikrobiziden Mittel nach Anspruch 6 als Algen- und Schleimbekämpfungsmittel.

9. Verwendung der mikrobiziden Mittel der Formel

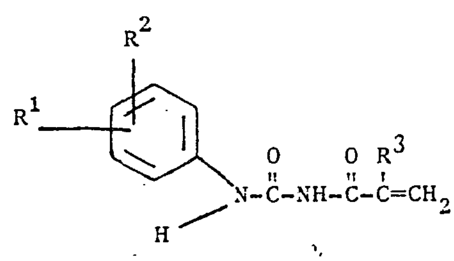

worin

$R^1$ und $R^2$ gleich oder verschieden sind und Wasserstoff, gegebenenfalls durch Halogen substituiertes Alkyl oder Halogen bedeuten, und

$R^3$ Wasserstoff oder ein geradkettiger oder verzweigter Alkylrest bedeutet, im Pflanzenschutz.

10. N-Aryl-N'-acryloyl-ureide der Formel

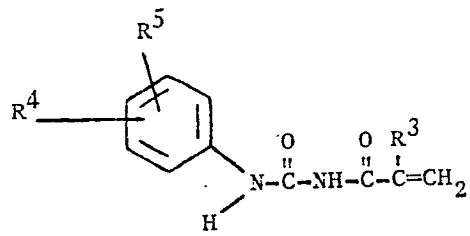

worin

$R^4$ und $R^5$ gleich oder verschieden sind und gegebenenfalls durch Halogen substituiertes Alkyl oder Halogen bedeuten, wobei für den Fall, daß eines von $R^4$ und $R^5$ Methyl bedeutet, das andere von $R^4$ und $R^5$ von Methyl verschieden ist, und

$R^3$ Wasserstoff oder ein geradkettiger oder verzweigter Alkylrest bedeutet.

**Claims** (for the Contracting States : DE, GB, FR)

1. Process for the preparation of N-aryl-N'-acrylylureides of the formula

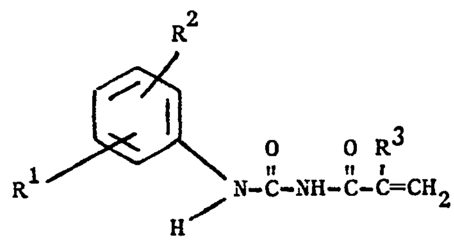

wherein

$R^1$ and $R^2$ are identical or different and denote hydrogen, alkyl which is optionally substituted by halogèn, or halogen and

$R^3$ denotes hydrogen or a straight-chain or branched alkyl radical, characterised in that arylureas of the formula

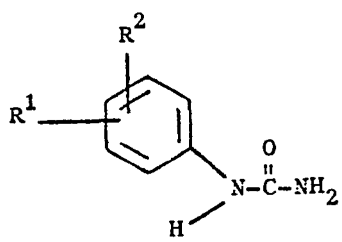

wherein

$R^1$ and $R^2$ have the abovementioned meaning, are reacted with a β-chloro-propionyl chloride of the formula

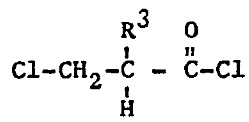

wherein

$R^3$ has the abovementioned meaning, and the N-aryl-N'-β-chloropropionyl-ureide formed is subsequently dehydrohalogenated with a base at elevated temperature.

2. Process according to Claim 1, characterised in that an arylurea of the formula

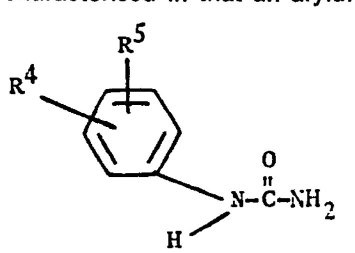

wherein

$R^4$ and $R^5$ are identical or different and denote alkyl which is optionally substituted by halogen, or halogen, and β-chloro-propionyl chloride are used as the starting substances.

3. Process according to Claims 1 and 2, characterised in that a tertiary amine is used as the base.

4. Microbicidal agent containing compounds of the formula

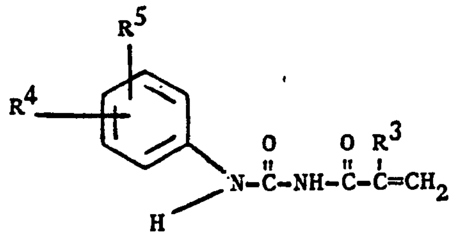

wherein

$R^3$ denotes hydrogen or a straight-chain or branched alkyl radical and

$R^4$ and $R^5$ are identical or different and denote alkyl which is optionally substituted by halogen, or halogen, wherein, in the case where one of $R^4$ and $R^5$ denotes methyl the other one of $R^4$ and $R^5$ is a radical other than methyl.

5. Use of the microbicidal agents of the formula

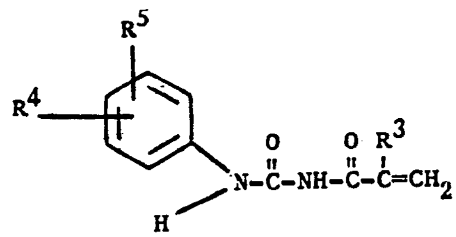

wherein

$R^3$ denotes hydrogen or a straight-chain or branched alkyl radical and

$R^4$ and $R^5$ are identical or different and denote alkyl which is optionally substituted by halogen, or halogen, wherein, in the case where one of $R^4$ and $R^5$ denotes methyl the other one of $R^4$ and $R^5$ is a radical other than methyl, for the protection of adhesives, sizes, papers and cardboards, textiles, leather, wood, paints, plasters and vessel contents which can be damaged or destroyed by microbial action.

6. Use of the microbicidal agents according to Claim 5 for protecting wood from microbial degradation.

7. Use of the microbicidal agents according to Claim 5 as agents for combating algae and slimes.

8. Use of the microbicidal agents of the formula

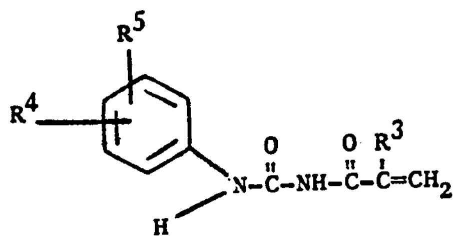

wherein

$R^3$ denotes hydrogen or a straight-chain or branched alkyl radical and

$R^4$ and $R^5$ are identical or different and denote alkyl which is optionally substituted by halogen, or halogen, wherein, in the case where one of $R^4$ and $R^5$ denotes methyl the other one of $R^4$ and $R^5$ is a radical other than methyl, in plant protection.

9. N-Aryl-N′-acrylyl-ureides of the formula

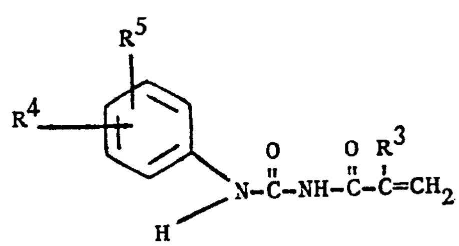

wherein

$R^4$ and $R^5$ are identical or different and denote alkyl which is optionally substituted by halogen or halogen, wherein in the case where one of $R^4$ and $R^5$ denotes methyl, the other one of $R^4$ and $R^5$ is a radical other than methyl, and

$R^3$ denotes hydrogen or a straight-chain or branched alkyl radical.

**Claims** (for the Contracting States : AT, BE, CH, IT, LI, SE)

1. Process for the preparation of N-aryl-N'-acrylylureides of the formula

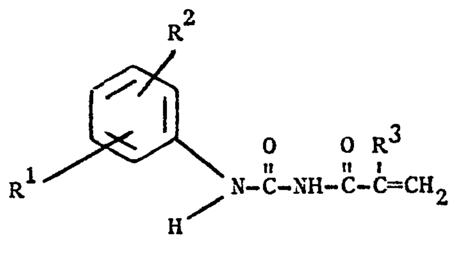

wherein

$R^1$ and $R^2$ are identical or different and denote hydrogen, alkyl which is optionally substitued by halogen, or halogen and

$R^3$ denotes hydrogen or a straight-chain or branched alkyl radical, characterised in that arylureas of the formula

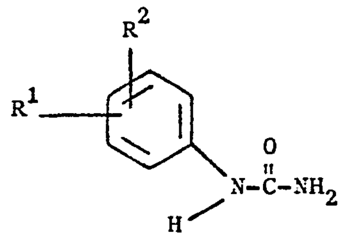

wherein

$R^1$ and $R^2$ have the abovementioned meaning, are reacted with a β-chloro-propionyl chloride of the formula

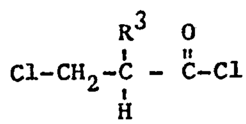

wherein

$R^3$ has the abovementioned meaning, and the N-aryl-N'-β-chloropropionyl-ureide formed is subsequently dehydrohalogenated with a base at elevated temperature.

2. Process according to Claim 1, characterised in that an arylurea of the formula

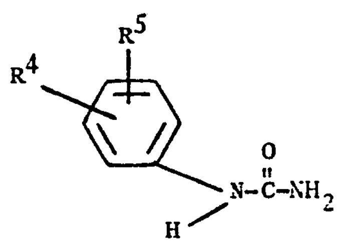

wherein

$R^4$ and $R^5$ are identical or different and denote alkyl which is optionally substituted by halogen, or halogen, and β-chloro-propionyl chloride are used as the starting substances.

3. Process according to Claims 1 and 2, characterised in that a tertiary amine is used as the base.

4. Microbicidal agent containing compounds of the formula

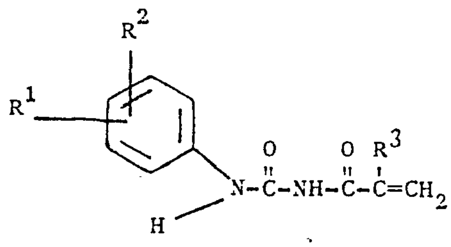

wherein

$R^1$ and $R^2$ are identical or different and denote hydrogen, alkyl which is optionally substituted by halogen, or halogen, wherein in the case where one of $R^1$ and $R^2$ denotes hydrogen, methyl or chlorine situated in the 4-position, the other one of $R^1$ and $R^2$ is always a radical other than hydrogen and wherein in the case where one of $R^1$ and $R^2$ denotes methyl, the other one of $R^1$ and $R^2$ also is a radical other than methyl, and

$R^3$ denotes hydrogen or a straight-chain or branched alkyl radical.

5. Microbicidal agent according to Claim 4, containing compounds of the formula

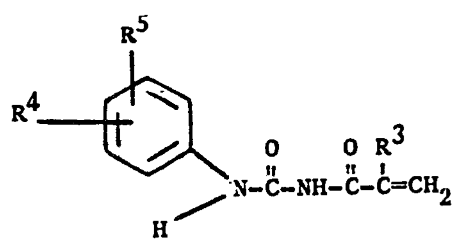

wherein

$R^3$ denotes hydrogen or a straight-chain or branched alkyl radical, and

$R^4$ and $R^5$ are identical or different and denote alkyl which is optionally substituted by halogen, or halogen, wherein in the case where one of $R^4$ and $R^5$ denotes methyl the other one of $R^4$ and $R^5$ is a radical other than methyl.

6. Use of the microbicidal agents of the formula

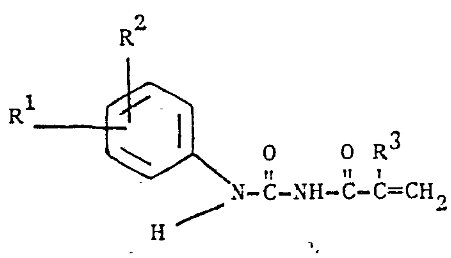

wherein

$R^1$ and $R^2$ are identical or different and denote hydrogen, alkyl which is optionally substituted by halogen, or halogen, and

$R^3$ denotes hydrogen or a straight-chain or branched alkyl radical, for the protection of adhesives, sizes, papers and cardboards, textiles, leather, wood, paints, plasters and vessel contents which can be damaged or destroyed by microbial action.

7. Use of the microbicidal agents according to Claim 6 for protecting wood from microbial degradation.

8. Use of the microbicidal agents according to Claim 6 as agents for combating algae and slimes.

9. Use of the microbicidal agents of the formula

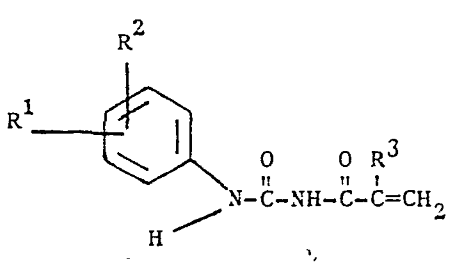

in which

$R^1$ and $R^2$ are identical or different and denote hydrogen, alkyl which is optionally substituted by

halogen, or halogen, and

$R^3$ denotes hydrogen or a straight-chain or branched alkyl radical, in plant protection.

10. N-Aryl-N'-acrylyl-ureides of the formula

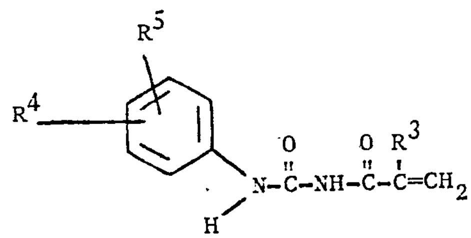

wherein

$R^4$ and $R^5$ are identical or different and denote alkyl which is optionally substituted by halogen, or halogen, wherein in the case where one of $R^4$ and $R^5$ denotes methyl, the other one of $R^4$ and $R^5$ is a radical other than methyl, and

$R^3$ denotes hydrogen or a straight-chain or branched alkyl radical.

**Revendications** (pour les Etats contractants : DE, GB, FR)

1. Procédé de préparation de N-aryl-N'-acryloyluréides de formule

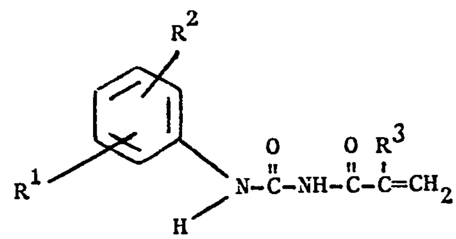

dans laquelle

$R^1$ et $R^2$ sont identiques ou différents et représentent chacun un atome d'hydrogène, un groupe alkyle éventuellement substitué par un atome d'halogène, ou encore un atome d'halogène, et

$R^3$ représente un atome d'hydrogène ou un groupe alkyle à chaîne droite ou ramifiée, caractérisé en ce qu'on fait réagir des arylurées de formule

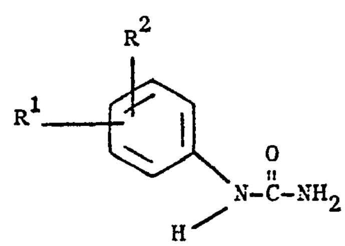

dans laquelle

$R^1$ and $R^2$ ont les significations indiquées ci-dessus, avec un chlorure de β-chloropropionyle de formule

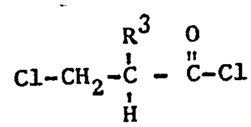

dans laquelle

$R^3$ a la signification indiquée ci-dessus, puis on soumet le N-aryl-N'-β-chloropropionyluréide formé à une déshydrohalogénation avec une base et à température élevée.

2. Procédé suivant la revendication 1, caractérisé en ce que, comme substances de départ, on utilise une arylurée de formule

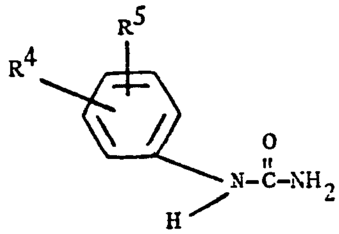

dans laquelle

$R^4$ et $R^5$ sont identiques ou différents et représentent chacun un groupe alkyle éventuellement substitué par un atome d'halogène, ou encore un atome d'halogène, et le chlorure de β-chloropropionyle.

3. Procédé suivant les revendications 1 et 2, caractérisé en ce que, comme base, on utilise une amine tertiaire.

4. Agent microbicide contenant des composés de formule

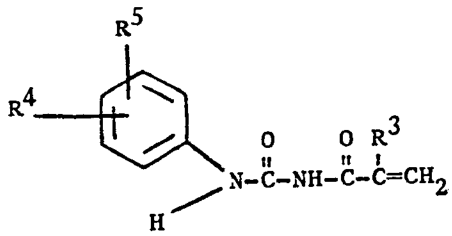

dans laquelle

$R^3$ représente un atome d'hydrogène ou un groupe alkyle à chaîne droite ou ramifiée, et

$R^4$ et $R^5$ sont identiques ou différents et représentent chacun un groupe alkyle éventuellement substitué par un atome d'halogène, ou encore un atome d'halogène, et, si un des radicaux $R^4$ et $R^5$ représente un groupe méthyle, l'autre est différent du groupe méthyle.

5. Utilisation d'agents microbicides de formule

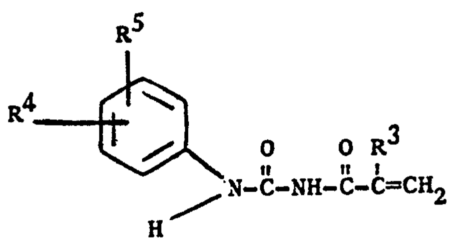

dans laquelle

$R^3$ représente un atome d'hydrogène ou un groupe alkyle à chaîne droite ou ramifiée, et

$R^4$ et $R^5$ sont identiques ou différents et représentent chacun un groupe alkyle éventuellement substitué par un atome d'halogène, ou encore un atome d'halogène et, si un des radicaux $R^4$ et $R^5$ représente un groupe méthyle, l'autre est différent du groupe méthyle, pour la protection des adhésifs, des colles, des papiers et des cartons, des matières textiles, du cuir, du bois, des peintures, des enduits et des contenus d'emballages en fûts qui peuvent être détériorés ou détruits par action microbienne.

6. Utilisation des agents microbicides suivant la revendication 5 pour la protection du bois contre la décomposition microbienne.

7. Utilisation des agents microbicides suivant la revendication 5 pour combattre les algues et les mucilages.

8. Utilisation des agents microbicides de formule

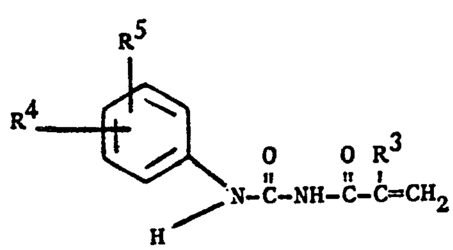

dans laquelle

$R^3$ représente un atome d'hydrogène ou un groupe alkyle à chaîne droite ou ramifiée, et

$R^4$ et $R^5$ sont identiques ou différents et représentent chacun un groupe alkyle éventuellement substitué par un atome d'halogène, ou encore un atome d'halogène et, si un des radicaux $R^4$ et $R^5$ représente un groupe méthyle, l'autre est différent du groupe méthyle, pour la protection des plantes.

9. N-aryl-N'-acryloyl-uréides de formule

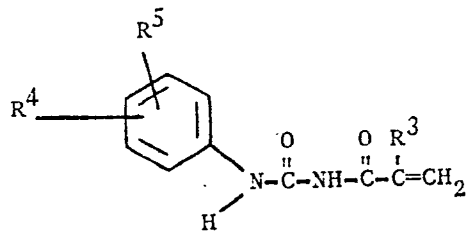

dans laquelle

$R^4$ et $R^5$ sont identiques ou différents et représentent chacun un groupe alkyle éventuellement substitué par un atome d'halogène, ou encore un atome d'halogène et, si un des radicaux $R^4$ et $R^5$ représente un groupe méthyle, l'autre est différent du groupe méthyle, et

$R^3$ représente un atome d'hydrogène ou un groupe alkyle à chaîne droite ou ramifiée.

**Revendications** (pour les Etats contractants : AT, BE, CH, IT, LI, SE)

1. Procédé de préparation de N-aryl-N'-acryloyluréides de formule

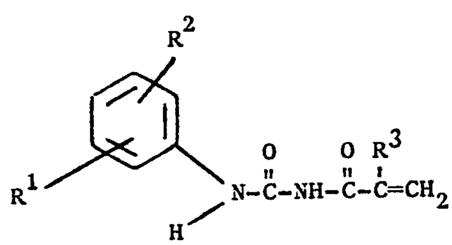

dans laquelle

$R^1$ et $R^2$ sont identiques ou différents et représentent chacun un atome d'hydrogène, un groupe alkyle éventuellement substitué par un atome d'halogène, ou encore un atome d'halogène, et

$R^3$ représente un atome d'hydrogène ou un groupe alkyle à chaîne droite ou ramifiée, caractérisé en ce qu'on fait réagir des arylurées de formule

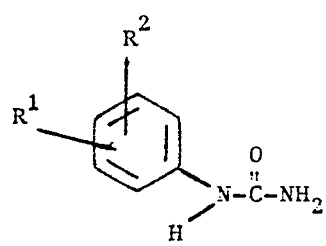

dans laquelle

$R^1$ et $R^2$ ont les significations indiquées ci-dessus, avec un chlorure de β-chloropropionyle de formule

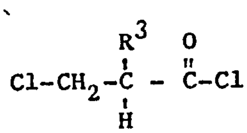

dans laquelle

$R^3$ a la signification indiquée ci-dessus, puis on soumet le N-aryl-N'-β-chloropropionyluréide formé à une déshydrohalogénation avec une base et à température élevée.

2. Procédé suivant la revendication 1, caractérisé en ce que, comme substances de départ, on utilise une arylurée de formule

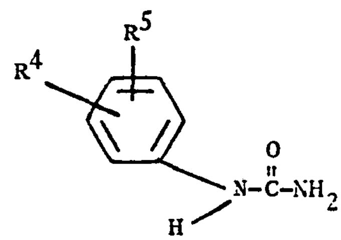

dans laquelle

$R^4$ et $R^5$ sont identiques ou différents et représentent un groupe alkyle éventuellement substitué par un atome d'halogène, ou encore un atome d'halogène, et le chlorure de β-chloropropionyle.

3. Procédé suivant les revendications 1 et 2, caractérisé en ce que, comme base, on utilise une amine tertiaire.

4. Agent microbicide contenant des composés de formule

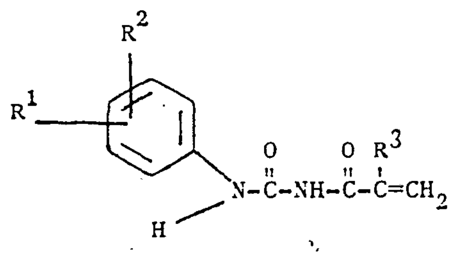

dans laquelle

$R^1$ et $R^2$ sont identiques ou différents et représentent chacun un atome d'hydrogène, un groupe alkyle éventuellement substitué par un atome d'halogène, ou encore un atome d'halogène et, si un des radicaux $R^1$ et $R^2$ représente un atome d'hydrogène, un groupe méthyle ou un atome de chlore en position 4, l'autre radical est toujours différent de l'hydrogène, tandis que, si un des radicaux $R^1$ et $R^2$ est un groupe méthyle, l'autre est également différent du groupe méthyle, et

$R^3$ représente un atome d'hydrogène ou un groupe alkyle à chaîne droite ou ramifiée.

5. Agent microbicide suivant la revendication 4 contenant des composés de formule

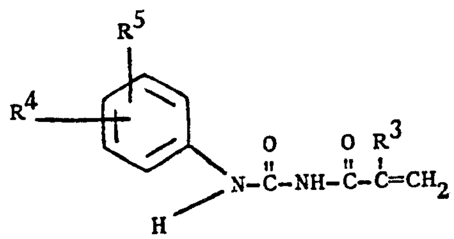

dans laquelle

$R^3$ représente un atome d'hydrogène ou un groupe alkyle à chaîne droite ou ramifiée, et

$R^4$ et $R^5$ sont identiques ou différents et représentent chacun un groupe alkyle éventuellement substitué par un atome d'halogène, ou encore un atome d'halogène et, si un des radicaux $R^4$ et $R^5$ représente un groupe méthyle, l'autre est différent du groupe méthyle.

6. Utilisation d'agents microbicides de formule

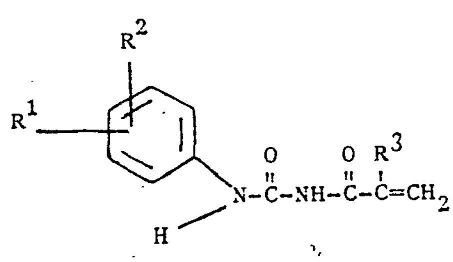

dans laquelle

$R^1$ et $R^2$ sont identiques ou différents et représentent chacun un atome d'hydrogène, un groupe alkyle éventuellement substitué par un atome d'halogène, ou encore un atome d'halogène, et

$R^3$ représente un atome d'hydrogène ou un groupe alkyle à chaîne droite ou ramifiée, pour la protection des adhésifs, des colles, des papiers et des cartons, des matières textiles, du cuir, du bois, des

peintures, des enduits et des contenus d'emballages en fûts qui peuvent être détériorés ou détruits par action microbienne.

7. Utilisation des agents microbicides suivant la revendication 6 pour protéger le bois contre la décomposition microbienne.

8. Utilisation des agents microbicides suivant la revendication 6 pour combattre les algues et les mucilages.

9. Utilisation des agents microbicides de formule

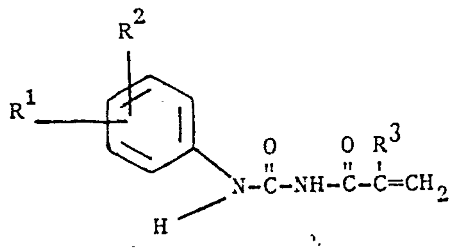

dans laquelle

$R^1$ et $R^2$ sont identiques ou différents et représentent chacun un atome d'hydrogène, un groupe alkyle éventuellement substitué par un atome d'halogène, ou encore un atome d'halogène, et

$R^3$ représente un atome d'hydrogène ou un groupe alkyle à chaîne droite ou ramifiée, pour la protection des plantes.

10. N-aryl-N'-acryloyluréides de formule

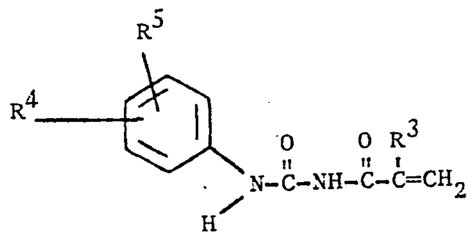

dans laquelle

$R^4$ et $R^5$ sont identiques ou différents et représentent chacun un groupe alkyle éventuellement substitué par un atome d'halogène, ou encore un atome d'halogène et, si un des radicaux $R^4$ et $R^5$ représente un groupe méthyle, l'autre est différent du groupe méthyle, et

$R^3$ représente un atome d'hydrogène ou un groupe alkyle à chaîne droite ou ramifiée.